Europäisches Patentamt

European Patent Office

Office européen des brevets

(11) Publication number: **0 244 386**
A2

## (12) EUROPEAN PATENT APPLICATION

(21) Application number: 87850145.1

(22) Date of filing: 30.04.87

(51) Int. Cl.³: **H 04 L 11/16**
    H 04 L 11/14

(30) Priority: 30.04.86 SE 8602021

(43) Date of publication of application:
    04.11.87 Bulletin 87/45

(84) Designated Contracting States:
    AT BE DE FR GB IT NL

(71) Applicant: UPEC ELEKTRONIK AB
    Sjöängsvägen 1 C
    S-191 72 Sollentuna(SE)

(72) Inventor: Ek, Claes Wilhelm
    Birkavägen 17
    S-198 00 Balsta(SE)

(74) Representative: Onn, Thorsten et al,
    AB STOCKHOLMS PATENTBYRA Box 3129
    S-103 62 Stockholm(SE)

(54) A device for transmitting digital information via a long distance transmission medium.

(57) A device for transmission of measured data in unmodulated form from a number of measuring units to a centre or between the measuring units. The transmission medium between the central unit (CE) and the different measuring units (KE1–KEn) consists of a cable with two phases (f1, f2) and a return conductor (r). Local voltage sources (D1, D2, C) are formed along the cable (KK) by feeding from for instance the central unit. The information in unmodulated form between the measuring units or between a measuring unit and the central unit is transmitted by connecting and disconnecting (S1, S2) the local voltage sources alternately and in dependence on the information to be transmitted. A galvanic isolation from the measuring units and lower distorsion in the transmission are obtained.

## FIG.8

EP 0 244 386 A2

## A device for transmission of digital information via a long distance transmission medium

### Technical field

This invention relates to a device according to the preamble of claim 1, especially for transmission of digital measurement signals from a number of transducers to a centre treating the transmitted measurement results. The device is also suitable for transmitting the digital measurement signals between the different communication units (transducers).

### State of art

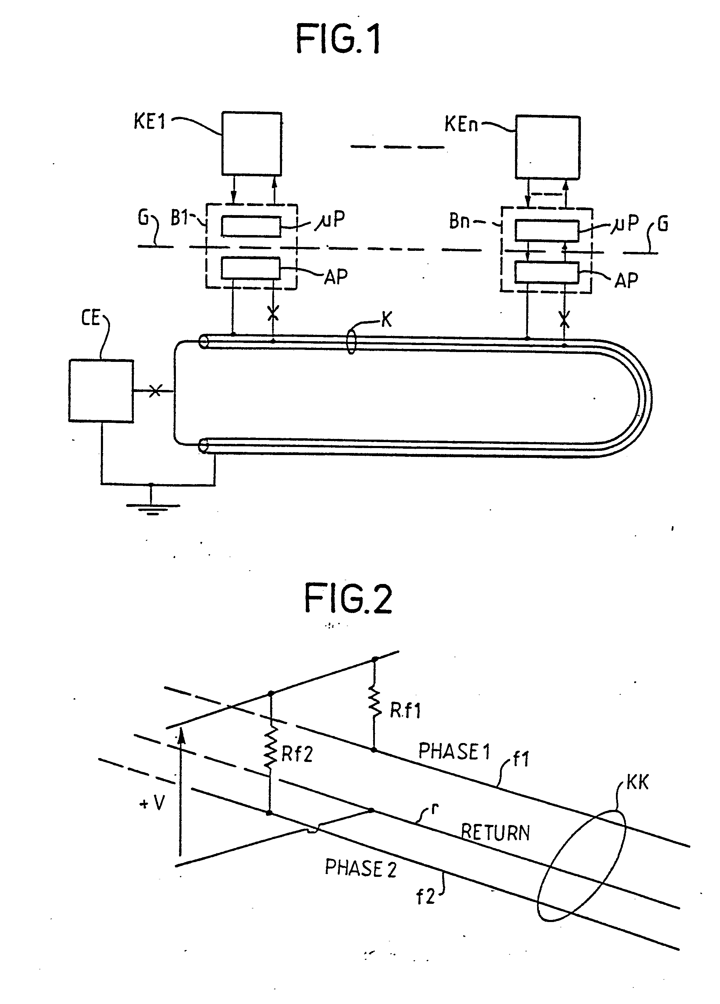

It is previously known, as above, to transmit physical measurement results in digital form from a number of communication units (transducers) to a centre and/or other communication units via a common transmission medium, for example a coaxial cable, in long distances. Fig. 1 in the enclosed drawings shows schematically an example of such a transmission. A central unit CE transmits and receives digital information in the form of pulsed signals ("0's and "1's") via a transmission medium, here in the form of a monoconductor cable K with interior conductor and screen. The cable K can also be a coaxial cable. A number of communication units KE1-KEn are connected to the cable via the blocks B1-Bn. In each block there is an adaptation unit AP and a calculating unit μP, for instance in the form of a microprocessor. The communication unit KE1, for example, consists of a water meter transmitting and receiving measurement data to the microprocessor in the block B1. The measuring signals are processed in the processor μP and thereafter forwarded to the adaptation unit AP. This unit transmits the measuring signals in the form of pulsed electric signals on the cable K to the central unit CE. It is necessary, in general, that the cable K and the central unit CE have a common reference potential (shown here as an earth potential) which is not shared by anyone of the communication units KE1-KEn. Therefore, in blocks B1-Bn there is an interface G galvanically separating the cable K from the communication units KE1-KEn.

## Summary of the invention

It is the object of this invention to solve the problem arising when it is intended to separate the transmission medium galvanically from the communication units. One way of solving this problem would be to arrange as interface G an electro-magnetic or optic coupling between the respective communication units and the cable K. However, in long-distance transmission this is not possible as the signals on the cable from the adaptation unit are generally too weak to be detected in for example the central unit CE from the communication unit KEn located furthest away. It is then necessary to arrange some form of feeding of the circuit located on the cable side to amplify the signal sent and transmitted by the cable. Is is true that magnetic coupling between the processing unit $\mu$P and the adaptation unit does not require any power feeding on the cable side but merely magnetic coupling of the input signals to the cable side without any amplification should not give acceptable results due to too great attenuation in the cable over long distances. Moreover, there will be a non-acceptable distorsion and delay of the pulse signals in the cable. The different changes from the 1-state to the 0-state and from the 0-state to the 1-state are especially delayed by different amounts.

It is the object of the invention to provide a transmission medium for digital signals between a number of communication units and a central unit or between merely the communication units, said transmission medium not suffering from the above-mentioned disadvantages or shortcomings in any high degree.

This problem is solved according to the invention in such a way that the transmission medium between the central unit and the different communication units is formed as a two-phase line with return conductors. and that local voltage sources are created along the line by feeding from for example a central unit, said voltage sources being galvanically isolated from the communication units. The information between the different communication units or between a communication unit and the central unit is transmitted by connecting and disconnecting the local voltage sources alternately and at the rate of the information to be transmitted.

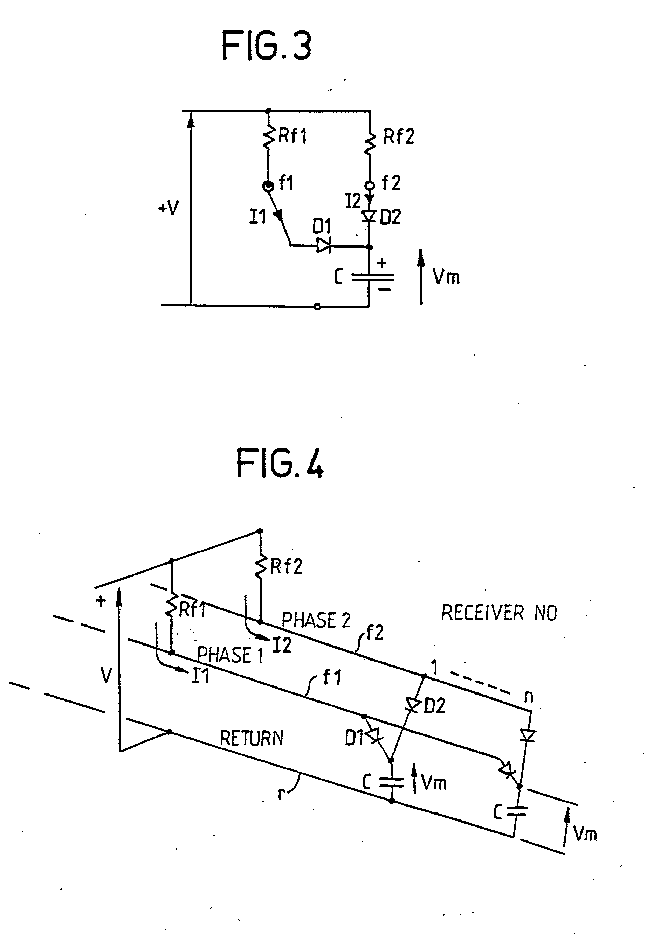

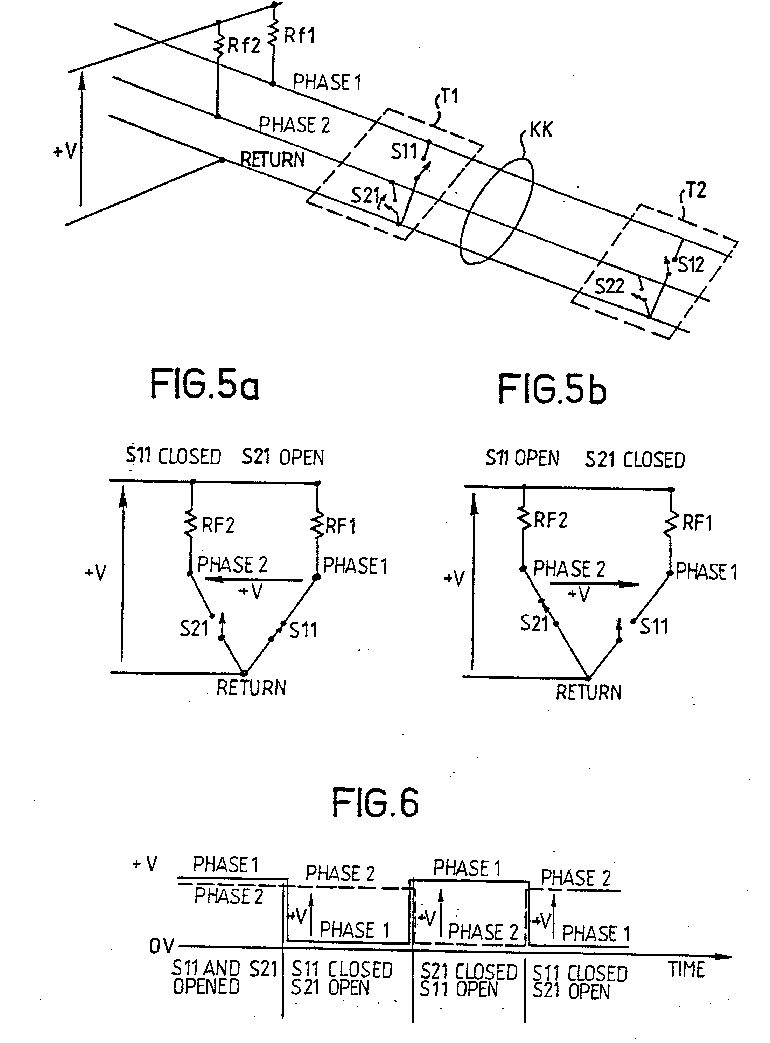

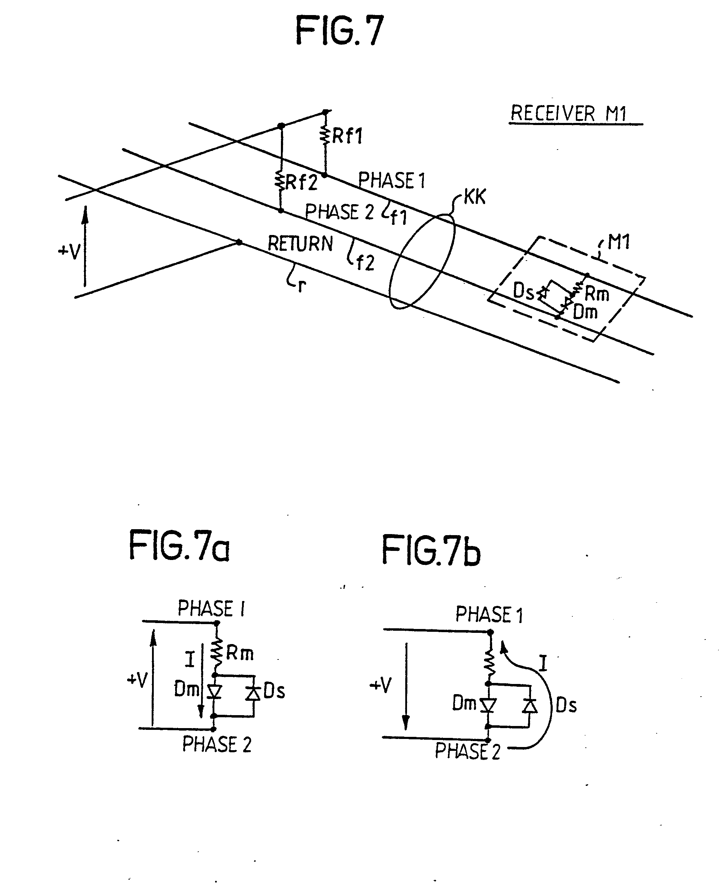

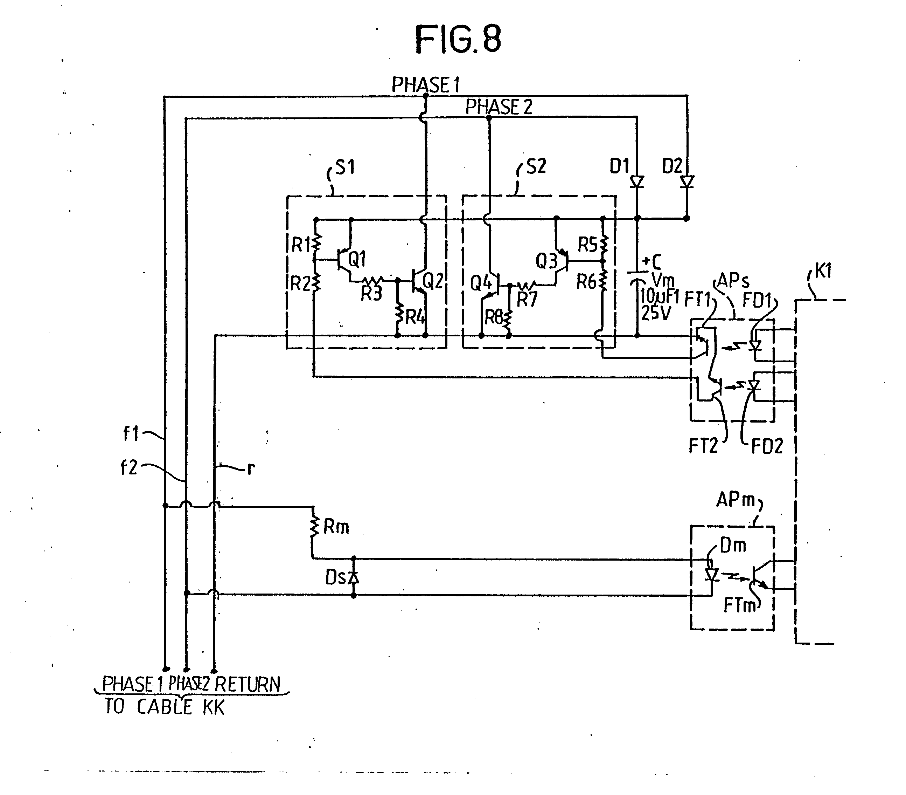

The invention, the characteristic features of which appear from the following claims will now be described more in detail with reference to the enclosed drawings, wherein Fig. 1 shows schematically a block diagram of a previously known information transmission between a number of communication units and a central unit via a cable, Fig. 2 shows a scheme of the cable included in the device of the invention, Fig. 3 shows a circuit diagram for illustrating connection of local voltage sources in the cable included in the invention, Fig. 4 is a combination of what is shown in Figs. 2 and 3, Fig. 5 is a circuit diagram where local transmitters in the form of switches are connected in the cable shown in Fig. 2, Figs. 5a and 5b show the different connection positions of the switches in Fig. 5, Fig. 6 shows a time-diagram of the switches according to Fig. 5, Fig. 7 shows connection of circuit elements in the cable according to Fig. 2 for reception, Figs. 7a, 7b show more closely circuit diagrams of the circuit elements according to Fig. 7, Fig. 8 shows a transmission side and a receiving side of a communication unit connected to the cable according to Fig. 2, Figs. 9 and 10 are circuit diagrams of the cable according to Figs. 7, 7a, 7b for two different switch cases to explain the advantages of the invention more closely.

## Description of a preferred embodiment

The appearance of the communication cable KK is shown more in detail in Fig. 2. The cable KK consists of three conductors f1, f2 and r. The conductors can either be enclosed in a separate three-way cable or comprise three of the conductors in a multi-conductor cable. No demands for screening, twisted pairs and so on are present. As to the area of the cable as well as the cross-sectional area of the different conductors these are related to the transmission paths in known manner. Two conductors f1, f2 of the three conductors f1, f2 and r are used to create two electrically identical phases, phase 1 and phase 2. The third conductor r is a common return conductor for the conductors of the two phases.

Phase 1 and phase 2 are created by connecting a voltage source V to f1, f2 via two identical resistors Rf1 and Rf2, respectively, at an arbitrary place along the cable. The return conductor r is connected to the zero potential of the voltage source. The voltage source V as well as its associated zero potential can be placed in the central unit CE or somewhere along the cable line.

When no current is flowing through the phase conductors f1, f2 and thus not through Rf1 or Rf2, either, the phase 1 and the phase 2 are maintained at the voltage V relative to the return conductor r via Rf1 and Rf2.

Fig. 3 shows how local voltage sources are created along the cable KK according to Fig. 2. If the anodes of two diodes D1 and D2 are connected at an arbitrary point along the cable, one to each phase f1, f2 according to Fig. 3 and the cathodes are connected in common to the positive plate of an electrolyte capacitor C, the other plate of which is connected to the return conductor r, currents I1 and I2 will flow from the voltage source V through the resistors Rf1 and Rf2,

respectively, through diodes D1 and D2. The capacitor C
will be successively charged to a final voltage equal
to V reduced by the forward voltage drop of the diodes
(of the order of 0.5 V). The resulting final voltage over
the capacitor is designated Vm.

If an optional number of similar diode pairs and
capacitors are connected to arbitrary points along the
cable KK according to Fig. 4 in a way shown in Fig. 3 all
capacitors C will be successively charged to a final
voltage Vm, after which a stationary state appears where
no currents flow. In this way a number of local voltage
sources having the voltage Vm have been created which have
all been charged via the single voltage source V.

In Fig. 5 the cable KK is shown again schematically
with switches connected to form transmission units associated with each of the communication units KE1-KEn. If
two switches S11 and S21 are connected to an arbitrary
point on the cable KK in such a way that S11 is connected
between phase 1 and return och S21 between phase 2 and
return and both switches remain in open position the
potential at the two phases is not actuated nor the
potentials Vm at the two voltage sources described in
connection with Fig. 4.

Figs. 5a and 5b are intended to show the voltage ratios
in the cable at different connection of the switches
S11 and S21. If the switches S11 are closed (see Fig. 5a)
a current will flow from the voltage source V through the
resistor Rf1, further through phase 1 up to the switch S11
and back to the zero pole of the voltage source. If the
resistance in the conductors f1, f2 and r included in the
cable KK is negligible as compared with the resistance Rf1
the voltage at phase 1 will approximately assume zero
potential (the potential of the return conductor) whereas
phase 2 remains at +V relative to phase 1. The voltages
Vm mentioned in connection with Fig. 4 are not actuated
since the diode D1 blocks current and prevents this from
flowing from the capacitors C to phase 1. Furthermore,
phase 2 retains the potentials Vm via D2.

If S11 is now opened and S21 closed (see Fig. 5b) phase
1 will instead be at the potential +V relative to phase 2,
while the voltage sources Vm are maintained unchanged in
accordance with the above description.

By closing S11 and S21 alternately the differential
voltage +V can thus be created between the two phases f1,
f2 where the voltage direction is reversed at the rate of
change of the switches S11 and S21, see the time diagram
according to Fig. 6.

If an optional number of similar switches S12 and S22
and so on are connected at arbitrary points along the
cable and all switches are maintained open the above prerequisites are not influenced according to Figs. 4, 5, 5a
and 5b.

The alternate potential difference +V between phase 1
and 2 can thereafter be achieved by activating an arbitrary
switch pair, for example S11, S21, of the pairs connected
to the cable according to the above if the other switch
pairs S12, S22 etc. remain open. All the switch pairs are
assumed to be open in the starting position.

Each of the switch pairs shown in Fig. 5 are now called
transmitters or transmitting units T1. A transmitter is
said to be in position of rest when both the switches are
open, i.e. when both phases f1 and f2 have the potential
V relative to the return conductor r. An optional number
of transmitters as above are connected on arbitrary points
along the cable.

A transmitter forwards information from a communication
unit KE1 of a kind known per se and shown in Fig. 1 and
which is normally connected to a local voltage source
220 VAC. In order to prevent problems with differences in earth
potential, which may arise if several communication
units KE1-KEn are connected along a very long cable, as
shown in Fig. 1, the information is transmitted for
example from the communication unit KEn to the transmitter T1 via two optoswitches, where the latter control
the switches S11 and S21, respectively, in the transmitter without any electric connection between KEn and T1.

This will be shown more in detail in connection with Fig. 8.

However, the signals from the optoswitches must be amplified to control the switches S11 and S21. The transmitter T1 is therefore supplemented with a local voltage source Vm, as shown in connection with Fig. 4, which drives amplifying stages of the switches S11 and S21. Provided these switches are activated alternately, as previously described, at least one of the phases f1 and f2 will always have the potential +V and tend to maintain the local voltage source Vm, voltage feeding to the amplifiers being maintained.

Similarly, an optional number of communication units KE1-KEn can now be connected with their respective transmitters T1 with maintained galvanic isolation between the communication cable KK and the transmitters, on one hand, and, on the other hand, the respective communication units KE1-KEn. As described above, the respective communication unit KE1-KEn can further create via its transmitter T1 an alternate potential between the phases f1 and f2 along the cable, see the time diagram according to Fig. 6.

The above description treats merely transmission from a communication unit to another communication unit or to the central unit. It will also be described in the following how reception of signals over the cable KK to a receiving communication unit takes place. With reference to Fig. 7 the structure of the cable KK is now shown again with the transmitters omitted. If a diode Dm with a current limiting series resistance Rm is connected between the phases f1 and f2 at an arbitrary point of the cable KK no current will flow through the diode Dm in the position of rest, i.e. all the switches in the respective transmission units open where the potential will be equal to +V over both phase 1 and phase 2 relative to the return conductor r. If, however, an arbitrary communication unit KEn creates via its transmitter T1 the potential +V between phase 1 and phase 2 (by closing a switch S21), a current will flow through the diode Dm and its size will be decided by the series resistance Rm. If the

transmitters T1 now reverse the polarity so that phase 2 has the potential +V relative to phase 1 (by opening the switch F21 and closing the switch F12) the current is blocked through the diode Dm and no current will flow. If, on the other hand, another diode DS oppositely directed relative to the diode Dm is connected in parallel with Dm a current will again flow in the opposite direction with the same size as before and defined by the potential +V and the same series resistance Rm. The elements Rm, Ds and Dm connected as shown in Fig. 7 will be a receiver for the signals transmitted by the cable KK to one of the communication units, as will be described below. The above means that the receiver is a symmetrical load through which identical currents flow but in the opposite direction depending on which of the switches is activated. By this it will be achieved that the signal delay over long distances in the pulse signals will be the same when passing from 1 to 0 as when passing from 0 to 1.

Fig. 7a is intended to show the current and voltage ratio in a receiver means according to Fig. 7 when the transmitter is in one position and Fig. 7b is to show the conditions in the receiver means when the transmitter is in the other position, compare Figs. 5a and 5b, respectively. If an optional numer of similar diode pairs Dm and Ds as well as the resistance Rm are connected between the phases f1 and f2 on arbitrary points along the communication cable KK current will flow through all the diodes Dm if an arbitrary communication unit connects via a transmitter T1 the switch F21 so that the potential +V is created between phase 1 and phase 2, Fig. 7a. If instead the switch S11 is closed the potential +V is created between phase 2 and phase 1 and the current through all the diodes Dm ceases to flow and flows instead through all the diodes Ds in the opposite direction, Fig. 7b. Thus, an arbitrary communication unit will through a transmitter T1 connect and disconnect the current through all the diodes Dm along the communication cable in a predetermined pattern corresponding to the information that is described to be transmitted via the cable KK to the central unit or between the communication units.

In analogy with what has been described above one can now let the diode Dm be light-emitting diode in an optoswitch. At the rate of connection and disconnection of the current through the diode Dm the corresponding signal pattern can be transmitted in the receiver M1 thus created to a communication unit connected to the optoswitch but otherwise galvanically isolated from the communication cable KK.

The transmitting-receiving units T1 and M1, respectively, and their adaptation to a communication unit will be described more closely with reference to Fig. 8.

In Fig. 8 one of the communication units KE1-KEn is indicated by dashed lines and designated K1. The unit K1 has an adaptation unit for transmission designated APs and one for reception designated APm. The adaptation unit APs is connected on its output side to the two switching units S1 and S2 of the transmitter T1 and to the local voltage source consisting of the capacitor C and the diodes D1, D2, compare Figs. 4 and 5. The adaptation unit APs to the transmitting side of the communication unit K1 consists of an optoswitch containing the photo-diodes FD1 and FD2 as well as the photo-transistors FT1 and FT2.

The adaptation unit APm to the receiving side consists of the photo-transistor FTm and the photo-diode Dm, where the photo-diode Dm corresponds to the diode Dm shown in Figs. 7, 7a, 7b, the resistance Rm and the diode Ds (symmetry diode) shown in Fig. 8 corresponding to the same elements as in Fig. 7.

The switches S1 and S2 correspond for instance to S11 and S21 in Fig. 5 included in the transmitter T1. These consist here of transistor circuits. The switch S1 con-sists of the transistors Q1 and Q2, the voltage divider R1, R2 as well as the associated resistors R3, R4. Similarly, the switch S2 consists of the transistors Q3 and Q4, the voltage divider R5, R6 as well as the associated resistors R7, R8. The base of the transistor Q1 in the switch S1 is

connectedvia resistor R2 to the collector of the photo-transistor FT2, the collector of the transistor Q2 forming the output of the switch S1 connected to phase 1. The base of the transistor Q3 is in the same way connected to the collector of the photo-transistor FT1 via the resistor R6 in the voltage divider R5, R6. The emitters of the two photo-transistors FT1, FT2 are connected in common to the return line r. The two photo-diodes FD1, FD2 are alternately activated, i.e. when for instance a "1" is to be transmitted from the communication unit K1 the photo-diode FD1 is activated and emits light towards the photo-transistor FT1, while the photo-diode FD2 is not activated and, thus, put out. Conversely, if a logic "0" is to be emitted the photo-diode FR1 is not activated and, thus, put out while the photo-diode FD2 is activated and emits light towards the photo-transistor FT2.

When the photo-transistor FT1 is activated by receiving light from the photo-diode FD1, a current will flow through the resistors R5 and R6 from the voltage source (capacitor) C. Q3 will then receive base current and conduct. As a result, Q4 will conduct and phase 2 is connected to the return line r, i.e. S2 is closed. When the photo-diode FD2 is activated and emits light in the photo-transistor FT2 base current is supplied to the transistor Q1 via the resistor R2 and this transistor starts to conduct. The transistor Q2 will then also receive base current and start to conduct, i.e. the switch S1 becomes closed.

The receiving side of the communication unit K1 is connected to a photo-transistor FTm which is optically connected to the photo-diode Dm which, in turn, is galvanically connected via the resistor Rm to the two phases f1, f2. A symmetry diode Ds is connected in antiparallel with the photo-diode Dm. By connecting the diode Ds it is achieved that the receiver formsa symmetrical load for the transmitters so that identical currents flow in transmission and reception but in opposite directions depending on which of the switches is activated, as described above.

0244386

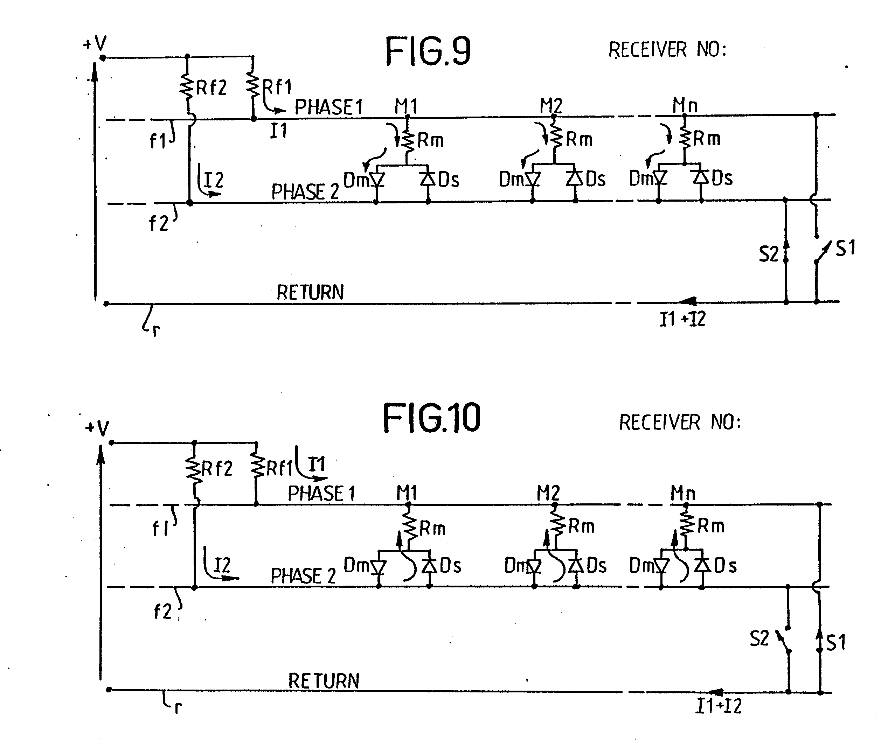

A brief analysis of the current conditions at transmission/reception will be given with reference to Figs. 9 and 10. Fig. 9 shows schematically the cable conductors f1, f2 and r as well as a transmitter (the switches S1 and S2), a number of receivers M1-Mn, and in the case when the switch S2 is closed and S1 is open. Fig. 10 shows the same structure but with the switch S1 closed and S2 open.

As previously shown, no currents flow through the cable KK in position of rest, i.e. when all the transmission switches S1 and S2 are open and when the potentials on the two phases f1 and f2 are +V.

.If the switch S2 is now closed in a transmitter T1 (compare Fig. 9) the phase 2 is short-circuited to return. The current I2

$$I2 = \frac{V}{Rf2} \qquad (1)$$

will then flow through the resistor RF2.

As to phase 1 the current is limited, besides the resistor Rf1, by n resistors Rm connected in parallel, where n is the number of receivers connected to the communication cable KK. The equivalent . resistance will therefore be Rm/n and the current

$$\cdot I_1 = V/(Rf1+Rm/n) = \frac{\frac{n \cdot V}{Rm}}{1 + \frac{n \cdot Rf1}{Rm}} \qquad (2)$$

The current I1 is superimposed on I2 and the current in the return conductor r is

$$I_{R2} = I_1 + I_2 = \frac{V}{Rf2} \dotplus \frac{\frac{n \cdot V}{Rm}}{1 + \frac{n \cdot Rf1}{Rm}} \qquad (3)$$

If the switch S2 is now opened and the switch S1 closed the currents according to Fig. 10 are obtained. Phase 1 will be on zero potential and the current will be

$$I_1 = \frac{V}{Rf1} \qquad (4)$$

In analogy with this the following applies to I2:

$$I_2 = \frac{V}{Rf2 + Rm/n} = \frac{\dfrac{n \cdot V}{Rm}}{1 + \dfrac{n \cdot Rf1}{Rm}} \qquad (5)$$

And the total current in the return conductor r will be

$$I_{R1} = I_1 + I_2 = \frac{V}{Rf1} + \frac{\dfrac{n \cdot V}{Rm}}{1 + n \cdot RM/Rm} \qquad (6)$$

To sum up, the current in the return conductor will be

SWITCH S2 CLOSED $$I_{R2} = \frac{V}{Rf2} + \frac{\dfrac{n \cdot V}{Rm}}{1 + \dfrac{n \cdot Rf1}{Rm}} \qquad (7)$$

SWITCH S1 CLOSED $$I_{R1} = \frac{V}{Rf1} + \frac{\dfrac{n \cdot V}{Rm}}{1 + \dfrac{n \cdot Rf2}{Rm}} \qquad (8)$$

If Rf1 = Rf2 = Rf, i.e Rf1 is selected to be equal to Rf2,

$$I_{R1} = I_{R2} = I_R = \frac{V}{Rf} + \frac{n \cdot V/Rm}{1 + n \cdot Rf1/Rm} \qquad (9)$$

is obtained, i.e. the size and direction of the current
in the return conductor r are independent of whether S1 or
S2 is closed. When transmitting a data pattern where S1
and S2 are closed alternately, the current in the return
conductor r is thus constant during the whole transmission. This means that the distorsion of the data signal
is reduced to a large extent.

## CLAIMS

1. A device for transmission of digital information via a long distance transmission medium between a number of transmitting and receiving communication units (KE1-KEn) or between a communication unit and a central unit (CE), all units being galvanically separated from the transmission medium (K), c h a r a c t e r i z e d in that the transmission medium comprises a cable (KK) consisting of three conductors, two signal transmitting conductors (f1,f2) of which are potential transmitting phases and the third is a reference return conductor (r) having a fixed potential, and that

a) a number of local voltage sources (D1,D2,C) are arranged along the cable (KK) between the respective phase (f1,f2) and fed via the cable from a common source (+V), which number is equal to the number of communication units (KE1-KEn) connected to the cable, that

b) a number of switches (S11,S12...,S21,S22) controlled from associated communication units are connected along the cable (KK) between the return conductor (r) and the respective phase (f1,f2) with a pair of switches (S11,S21) for each communication unit (KE1), said pair forming a transmitter for each such unit for sending out and transmitting pulse signals along the cable to the other units, and that

c) circuit elements (Rm,Dm) conductive in one direction are connected between the two phases (f1,f2) along the cable (KK) and for each communication unit, which are activated by said pulse signals upon activation of a transmitter in order to transmit these signals without galvanic coupling in the pattern defined by a transmitter to the associated receiving communication unit (KE1).

2. The device of claim 1, c h a r a c t e r i z e d in that each of said pair of switches (S11,S21;S12,S22) are controlled by means of a non-galvanic switch from the respective communication unit (KE1,KE2).

3. The device of claim 2, c h a r a c t e r i z e d in that the non-galvanic coupling to a pair of switches (S1,S2) consists of an optoswitch unit (APs) arranged between the pair of switches and the associated communication unit.

4. The device of claim 1, c h a r a c t e r i z e d in that a further circuit element ($D_s$) conductive in one direction is connected between the two phases (f1,f2), the conducting direction of which is opposite to the first-mentioned conductive circuit element (Dm).

5. The device of claim 1, c h a r a c t e r i z e d in that said local voltage sources each consist of a capacitor (C), one plate of which is connected to the return conductor (r) and to the two phases (f1,f2) via a first and a second diode (D4,D2) having the same conducting direction relative to the capacitor.

6. The device of claims 1-5, c h a r a c t e r i z e d in that the common source consists of a voltage source (+V) which is connected by one of its poles (+) to the two phases (f1,f2) and to the return line (r) by its other pole for charging the capacitor (C) to a final voltage (Vm).

7. The device of claim 6, c h a r a c t e r i z e d in that resistors ($R_{f1}$,$R_{f2}$) are connected between each phase (f1,f2) and one pole of the voltage source, said resistors being of the order of 100 Ohm.

8. The device of claim 1 or 4, c h a r a c t e r i z e d in that the first-mentioned conducting circuit element (Dm) is a light-emitting diode arranged to be optically connected to a photo-transistor (FTm) included in the associated communication unit (K1).

-----------

# FIG.1

# FIG.2

# FIG.3

# FIG.4

# FIG.5

Rf2  Rf1

PHASE 1

T1

PHASE 2

S11

KK

+V

RETURN

S21

T2

S12

S22

# FIG.5a

S11 CLOSED   S21 OPEN

RF2   RF1

PHASE 2   PHASE 1

+V

+V

S21   S11

RETURN

# FIG.5b

S11 OPEN   S21 CLOSED

RF2   RF1

PHASE 2   PHASE 1

+V

+V

S21   S11

RETURN

# FIG.6

+V  PHASE 1   PHASE 2   PHASE 1   PHASE 2

PHASE 2                                PHASE 2

+V  PHASE 1   +V  PHASE 2   +V  PHASE 1

0V

S11 AND S21 | S11 CLOSED | S21 CLOSED | S11 CLOSED
OPENED      | S21 OPEN   | S11 OPEN   | S21 OPEN

TIME

-4/6-

0244386

# FIG.7

RECEIVER M1

## FIG.7a          FIG.7b

FIG.8

0244386

FIG.9    RECEIVER NO:

FIG.10    RECEIVER NO: